# EUROPÄISCHE PATENTSCHRIFT

(11) **EP 0 611 548 B1**
(45) Veröffentlichungstag und Bekanntmachung des Hinweises auf die Patenterteilung: **09.05.2001**
(21) Anmeldenummer: 94101913.5
(22) Anmeldetag: 08.02.1994
(51) Int. Cl.: A61B 5/00, A61B 5/0448

(54) **Sensoreinrichtung zum Messen von vitalen Parametern eines Feten während der Geburt**
Sensor device adapted to measure vital data of a fetus during delivery
Capteur destiné à la mesure de paramètres vitaux d'un foetus pendant l'accouchement

(30) Priorität: 16.02.1993 DE 4304693
(43) Veröffentlichungstag der Anmeldung: 24.08.1994
(73) Patentinhaber: Rall, Gerhard, Dipl.-Ing., D-81545 München (DE); KNITZA, Reinhold, Dr., D-82131 Gauting (DE)
(72) Erfinder: Rall, Gerhard, Dipl.-Ing., D-81545 München (DE); KNITZA, Reinhold, Dr., D-82131 Gauting (DE)
(74) Vertreter: Grünecker, Kinkeldey, Stockmair & Schwanhäusser Anwaltssozietät

(56) Entgegenhaltungen:
- EP-A- 0 104 619
- EP-A- 0 135 840
- EP-A- 0 451 560
- DE-A- 2 619 471

## Beschreibung

Die Erfindung betrifft eine Sensoreinrichtung als Teil einer Meßvorrichtung mit einem Meßgerät zum Messen von vitalen Parametern eines Feten während der Geburt, insbesondere des Sauerstoffgehalts des Blutes des Feten, wobei die Sensoreinrichtung einen im Querschnitt etwa runden Träger mit einer Höhlung aufweist, in welcher ein Befestigungsmittel zum Befestigen des Trägers am führenden Teil des Feten angeordnet ist und der Träger als eine gekrümmte Schale ausgebildet ist, die auf ihrer konkaven Seite die Höhlung bildet.

Die Fachwelt bemüht sich bereits seit mehr als zehn Jahren intensiv, eine solche Sensoreinrichtung zum Überwachen des Sauerstoffgehalts im Blut des Feten zu schaffen, die während der Geburt sichere Signale zu einer zuverlässigen Beurteilung liefert, ob eine kindliche Notsituation vorliegt.

Bis heute ist dies jedoch nicht gelungen, so daß im In- und Ausland immer noch das in der Fachwelt als CTG-Verfahren bezeichnete Vorgehen (die Gebärende ist an einem Wehenschreiber angeschlossen und es wird die Herzfrequenz des Feten überwacht) angewandt wird, obwohl von der Fachwelt eingeräumt wird, daß dieses Verfahren häufig keine sicheren Erkenntnisse über eine kindliche Notsituation während der Geburt vermittelt. Die Folge ist bisher, daß im Zweifelsfall die Geburt operativ, insbesondere durch Kaiserschnitt, erfolgt. Dies erklärt, warum die Rate der Kaiserschnittgeburten in Deutschland erschreckend hoch ist, nämlich etwa 18 Prozent.

### Stand der Technik im einzelnen:

Bei einer aus der DE-A-26 19 471 bekannten Sensoreinrichtung der eingangs genannten Gattung hat der scheibenförmige Körper eine sehr flache Höhlung und besteht aus Material mit geringem Wärmeleitvermögen. An der konkaven Seite sind Temperatursensoren vorgesehen und es können dort auch Sensoren zum Messen des Sauerstoffgehalts des fetalen Bluts angebracht werden. Wenn die Sensoreinrichtung als EKG-Elektrode benutzt werden soll, so wird sie mittels einer Kontaktfeder in der Haut verankert.

Um einen besonders sicheren Sitz der Sensoreinrichtung zu gewährleisten sind an der konkaven Seite Mulden vorgesehen, die mit Klebstoff gefüllt werden. Dabei besteht die Gefahr, daß die Haut des Feten beim Bewegen des Feten oder der Mutter während der Geburt von den nicht mit Klebstoff versehenen Sonden abhebt, so daß keine zuverlässigen Meßsignale erhalten werden.

Eine andere Sensoreinrichtung ist durch die DE-PS 38 10 008, Fig. 5, bekannt geworden.

Dort ist ein Träger in Form eines Zylinders ausgebildet, an dessen einem Ende sich eine Höhlung mit einer beträchtlichen Tiefe befindet, aus welcher als Befestigungsmittel eine Drahtspirale verhältnismäßig weit herausragt.

Der Träger ist aus einem praktisch unnachgiebigen Werkstoff. Ein Lichtemitter und ein Empfänger sind gegenüberliegend nahe am Rand der Höhlung angebracht.

Mit der Spirale wird das Gewebe des Feten so weit in die Höhlung hineingezogen, daß sich dieses zwischen dem Lichtemitter und dem Empfänger befindet und so durchleuchtet werden kann.

Im Rahmen der Erfindung wurde festgestellt, daß durch das Hineinziehen des Gewebes in die Höhlung der arterielle Blutfluß in diesem Bereich beeinträchtigt werden kann, so daß keine verwertbaren Signale erzielt werden können.

Bei einer anderen bekannten Sensoreinrichtung (EP-OS 135 840, Fig.10 und 11) ist der Träger als Saugnapf aus Silicongummi ausgebildet. Auf der konkaven Seite des Saugnapfes sind Lichtemitter und Empfänger etwa gleich weit vom Zentrum des Saugnapfes und von seinem Rand entfernt angebracht. Der Saugnapf soll mittels einer Handhabe in Form einer Stange auf das Gewebe des Feten gedrückt und so die Luft aus der Höhlung des Saugnapfes ausgetrieben werden.

Eine sichere Befestigung des Saugnapfes am Gewebe des Feten während der Dauer der Geburt ist dabei nicht zu erreichen, dazu ist das erzielbare Vakuum zu schwach. Außerdem verhindern dies auch die regelmäßig auf dem Kopf des Feten befindlichen Härchen. Würde man den Saugnapf an eine Vakuumquelle anschließen, wie dies mit Rücksicht auf die Zeichnung Fig. 10 und 11 wegen der in der Wandung der Höhlung sternförmig angeordneten Schlitze für den Durchtritt von Luft angedeutet ist, würden bei einem schwachen Vakuum die oben geschilderten Schwierigkeiten ebenfalls auftreten. Würde aber ein deutlich höheres Vakuum angelegt, so würde durch den hohen Anpreßdruck der arterielle Fluß an der Meßstelle im Gewebe des Feten unterbrochen. Ein Anbringen des Trägers mittels Vakuum in der vorbekannten Art ist daher nicht erfolgversprechend. Auf der Seite 8 unten der EP-OS 0135 840 wird eine Befestigung des Sensors mittels einer Drahtspirale (sharp corkscrew) wegen hoher Risiken ausgeschlossen (Schaden am Hirn oder Auge sowie Infektionsgefahr).

Gemäß einer anderen Ausführungsform (Fig.4 bis 8) dieser Druckschrift weist der Sensor eine paddelförmige Gestalt auf, wobei ein paddelförmiges Gehäuse mit einem oberen Teil und einem unteren Teil vorgesehen ist, in welch letzterem eine zum Feten gewandte Höhlung zur Aufnahme von Lichtemitter und Empfänger vorhanden ist.

Der Sensor liegt mit dem oberen Teil am Gewebe des Feten an. Wenn die Höhlung mit einer Vakuumquelle verbunden wird, wird Gewebe in die Höhlung hineingezogen, so daß das Gewebe sich an Lichtemitter und Empfänger anlegt (siehe auch Fig. 15). Dabei wird der obere Teil des Gehäuses verhältnismäßig stark an das Gewebe angepreßt, was wiederum zu einer Beeinträchtigung des arteriellen Blutflusses führt.

Eine andere Ausbildung einer Sensoreinrichtung zum Überwachen des Sauerstoffgehalts eines Feten während der Geburt ist durch die EP-OS 0 104 619 angegeben.

Diese Sensoreinrichtung weist einen im Querschnitt runden Träger mit einer dem Feten zugewandten flachen Höhlung auf. Der Träger besteht aus Silikon-Plastik und zeigt etwa die Form eines Napfes von beträchtlicher Höhe.

In diesem Napf ist eine hohle Spiralnadel eingebettet, die aus der Höhlung zentrisch herausragt und als Befestigungsmittel dient.

Die Spiralnadel weist ein hohles schräg angeschärftes Ende auf. Im Abstand vom hohlen Ende ist in der Wandung der hohlen Spiralnadel ein Fenster angeordnet. Im Bereich desselben befindet sich im Inneren der hohlen Spiralnadel ein optischer Sensor, zu welchem zwei optische Fasern führen, die im Inneren der hohlen Spiralnadel angeordnet sind.

Das rückwärtige Ende der hohlen Spiralnadel ragt aus dem Träger seitlich heraus. Die optischen Fasern treten aus diesem rückwärtigen Ende aus und führen zu einer Lichtquelle und zu einem Empfänger.

Am Rande der flachen Höhlung sind mehrere halbrunde Erhebungen vorgesehen, so daß sich der Träger nur mit diesen Erhebungen am Gewebe des Feten abstützt, wenn der Träger mittels der Spiralnadel am Gewebe des Feten befestigt ist.

Dabei fließt arterielles Blut des Feten in das hohle Ende der Spiralnadel und wird dort durch den optischen Sensor kontrolliert.

Infolge dieses anderen Systems ergibt sich bei diesem bekannten Sensor nicht das Problem, daß infolge der Befestigung des Trägers der Blutfluß im Gewebe des Feten an der Meßstelle unterbrochen wird.

Auch spielt dort ein lichtdichtes Anliegen des Trägerrandes am Gewebe keine Rolle, so daß der infolge der Erhebungen sich einstellende Spalt zwischen dem Träger und dem Gewebe insoweit nicht schädlich ist.

Aufgabe der Erfindung ist es, eine Sensoreinrichtung der im Oberbegriff des Anspruchs 1 beschriebenen Art so auszubilden, daß gewährleistet ist, daß nach dem Anbringen des Sensors am Gewebe des Feten verwertbare Signale für die Dauer der Geburt erhalten werden.

Diese Aufgabe wird erfindungsgemäß gelöst durch eine Sensoreinrichtung mit den Merkmalen des Patentanspruchs 1.

Der Sensor wird durch die Maßnahmen gemäß der Erfindung also in zwei Zonen unterteilt, nämlich in eine Zentrumszone, in welcher das Befestigungsmittel den Sensor am Gewebe des Feten festhält, und in eine als Federmittel ausgebildet Randzone, die unter einer sanften Vorspannung auf dem Gewebe aufliegt, so daß der arterielle Blutfluß des Feten in dieser Zone auf keinen Fall beeinträchtigt wird. In dieser unproblematischen Randzone befinden sich nun Lichtemitter und Empfänger. Der Lichtemitter schickt das Licht in das Gewebe des Feten, wo es sich nach allen Richtungen ausbreitet und dabei ein Teil des Lichtes auf den Empfänger trifft.

Die Teilmerkmale des Anspruches 1 bilden eine Kombination und wirken zur Lösung der Aufgabe folgendermaßen zusammen: Infolge der zentrischen Festhaltekraft und der etwa runden Schalenform des Sensors wird die Randzone mit praktisch gleichmäßiger Vorspannung an das Gewebe des Feten angedrückt. Während die Befestigungszone mit dem Gewebe fest verbunden ist, d.h. keine Relativbewegungen zum Gewebe ausführen kann, liegt die elastische Randzone federnd, d.h. mit Vorspannung, auf dem Gewebe auf. Die Randzone dichtet die Höhlung des Sensors infolgedessen ungeachtet der Begungen des Feten während der Geburt luftdicht ab.

Dadurch, daß Lichtemitter und Empfänger in der Höhlung des Sensors in der elastischen Randzone angeordnet sind, werden sie durch die auf dem Gewebe mit Vorspannung aufliegende Randzone immer auf das Gewebe gedrückt. Das vom Emitter ausgehende Licht kann also nur durch das Gewebe zum Empfänger gelangen.

Das Teilmerkmal, daß die als Federmittel ausgebildete Randzone der am Gewebe befestigten Schale am Gewebe des Feten federnd nachgiebig aufliegt bedeutet, daß die Krümmung der Schale so groß ist, daß zu Beginn des Befestigungsvorganges als erstes die elastische Randzone des Sensors am Gewebe des Feten anliegt. Wenn während des Befestigungsvorganges schließlich auch die im Zentrum der gekrümmten Schale befindliche Befestigungszone am Gewebe anliegt, wird die Andrückkraft auf die elastische Randzone erhöht und so eine sichere luftdichte Abdichtung gewährleistet.

Von ganz besonderer Bedeutung ist, daß die als Federmittel ausgebildete Randzone der Schale federnd nachgiebig auf dem Gewebe aufliegt, und zwar im Hinblick auf das Aufrechterhalten des Blutflusses im Gewebe. Durch die Wahl der Vorspannung der Randzone im befestigten Zustand des Sensors kann eine Beeinträchtigung des Blutflusses mit Sicherheit vermieden werden, ohne daß die Gefahr besteht, daß der Sensor während der Geburt eine sichere Befestigung verliert. Gemäß der Erfindung kann das Befestigungsmittel als Drahtspirale derart angeordnet und ausgebildet sein, daß sie mit einem beliebig geformten Teil im Werkstoff der Schale eingebettet ist und der andere als Spirale ausgebildete Teil mit etwa einer Windung aus der Fläche der Höhlung ragt.

So kann durch eine Drehung des Trägers und damit auch der Spirale um etwa 360° eine sichere und dennoch schonende Befestigung der Schale am Gewebe des Feten erreicht werden.

Es kann das Befestigungsmittel aber auch durch einen Klebstoff dargestellt sein.

Für das Eindringen des Lichts in das Gewebe des Feten ist es vorteilhaft, wenn Lichtemitter und Empfänger zum Teil im Werkstoff der Schale eingebettet sind und ihre Oberfläche etwa bündig mit der Fläche der Höhlung verläuft.

Damit der Modulationsgrad des empfangenen Lichtes ein Höchstmaß erreicht, kann gemäß der Erfindung der Werkstoff der Schale eine Farbe aufweisen, die für die verwendeten Lichtwellen undurchlässig ist oder sie absorbiert.

Es ist aber auch in diesem Zusammenhang möglich, daß Lichtemitter und Empfänger innerhalb der Schale nach rückwärts abgeschirmt sind und die Fläche der Höhlung eine Farbe aufweist, welche die verwendeten Lichtwellen absorbiert.

Damit möglichst viel Licht seinen Weg durch das im Bereich der Randzone befindliche Gewebe des Feten zum Empfänger findet, sind vorteilhaft Lichtemitter und Empfänger zum Zentrum der Schale etwa gegenüberliegend, aber in einem Winkel von ungleich 180° angeordnet.

Eine besonders günstige Wirkung kann dabei dadurch erzielt werden, daß zwei Lichtemitter und ein Empfänger vorgesehen sind, die jeweils etwa auf einem Eckpunkt eines Dreiecks liegen. Dabei kann die Befestigungszone innerhalb dieses Dreiecks angeordnet sein.

Weitere Merkmale und Vorteile der Erfindung ergeben sich aus den Unteransprüchen sowie der nachfolgenden Beschreibung in Verbindung mit der Zeichnung, in welcher Ausführungsbeispiele der Erfindung dargestellt sind.

Dabei zeigen:
- Figur 1: in Draufsicht den am Kopf des Feten befestigten Träger (Sensor) in Verbindung mit dem Meßgerät, vergrößert,
- Figur 2: einen Axialschnitt durch den unverformten Träger, stark vergrößert,
- Figur 3: einen Axialschnitt durch den am Feten befestigten Träger, - ebenfalls stark vergrößert -,
- Figur 4: eine Untersicht des Trägers (Sensor) in einer ersten Ausgestaltung, - ebenfalls stark vergrößert -,
- Figur 5: eine Untersicht des Trägers (Sensor) in einer zweiten Ausgestaltung, ebenfalls stark vergrößert -, und
- Figur 6: eine Einzelheit VI in Figur 2 (sehr stark vergrößert).

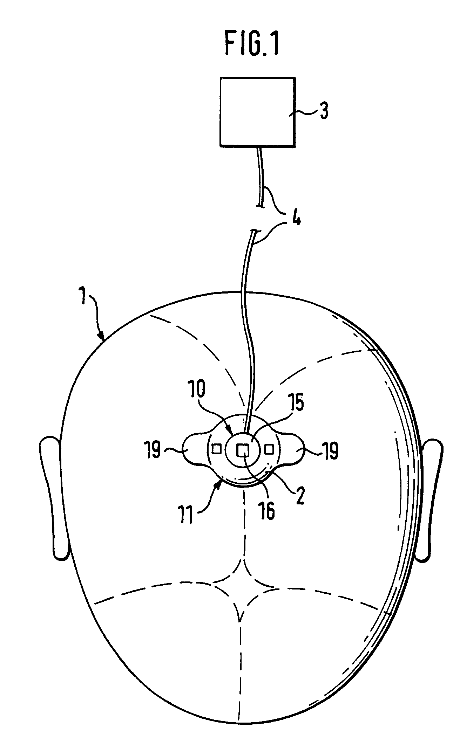

In Figur 1 ist der bei der Geburt führende Teil des Feten, hier der Kopf 1, zu sehen, wobei der Geburtsgang der Mutter aus Gründen der Übersicht weggelassen ist. Der Träger 2 (Sensor) ist durch die Vagina eingeführt und am Kopf 1 befestigt.

Der Sensor 2 ist mit dem Meßgerät 3 durch allgemein als 4 bezeichnete Leitungen verbunden.

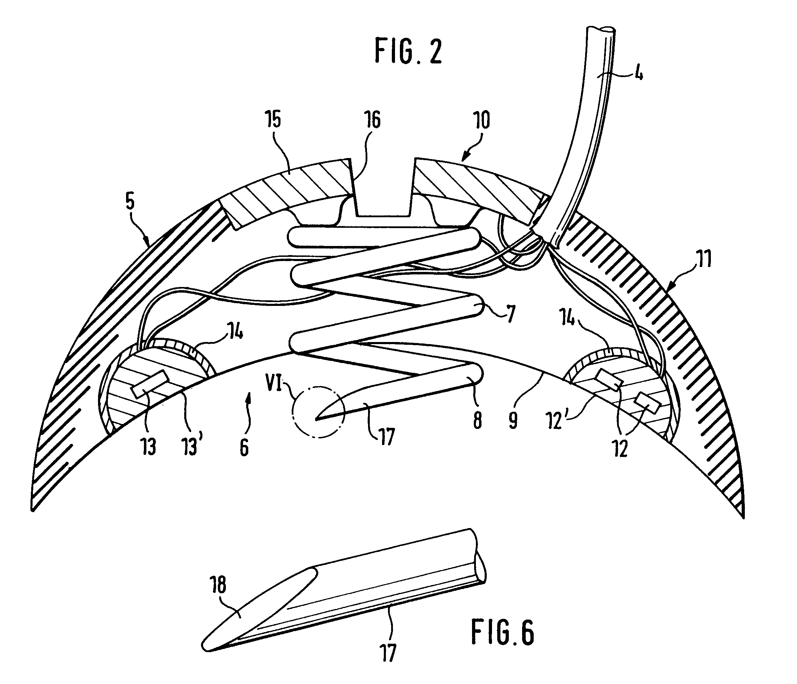

In Figur 2 ist der Träger 2 in einem ersten Ausführungsbeispiel gezeigt, ohne daß er am Kopf des Feten befestigt ist, also unverformt ist.

Der Träger 2 ist als eine, im Querschnitt gekrümmte runde Schale 5 ausgebildet, die sich zum Rand hin deutlich verjüngt. Die Schale 5 weist auf ihrer dem Kopf 1 des Feten zugewandten konkaven Seite eine Höhlung 6 auf.

Der Träger 2 besteht aus einem gummiähnlichen, verhältnismäßig weichen Werkstoff.

Im Zentrum der Schale 5 ist eine Drahtspirale 7 eingebettet, die mit etwa einer Windung 8 aus der Fläche 9 der Höhlung 6 ragt.

Der Träger weist somit eine im Zentrum angeordnete Befestigungszone 10 auf.

Sie ist umgeben von einer elastischen Randzone 11, wo ein Lichtemitter 12 und ein Empfänger 13 angeordnet sind, die im Werkstoff der Randzone 11 eingebettet sind, wobei ihre Oberfläche 12' und 13' etwa bündig mit der Fläche 9 der Höhlung 6 verläuft. Die Krümmung der Höhlung 6 der Schale 5 in unverformten Zustand ist größer als die statistisch größte Krümmung eines Feten-Kopfes.

Der Lichtemitter 12 und der Empfänger 13 ist jeweils innerhalb der Schale 5 nach rückwärts mittels einer Kappe 14 optisch abgeschirmt. Gleichzeitig weist die Fläche 9 der Höhlung 6 eine Farbe auf, die für die verwendeten Lichtwellen undurchlässig ist oder sie absorbiert.

Die gleiche Wirkung kann erzielt werden, wenn der Werkstoff der Schale 5 eine Farbe aufweist, welche die verwendeten Lichtwellen absorbiert.

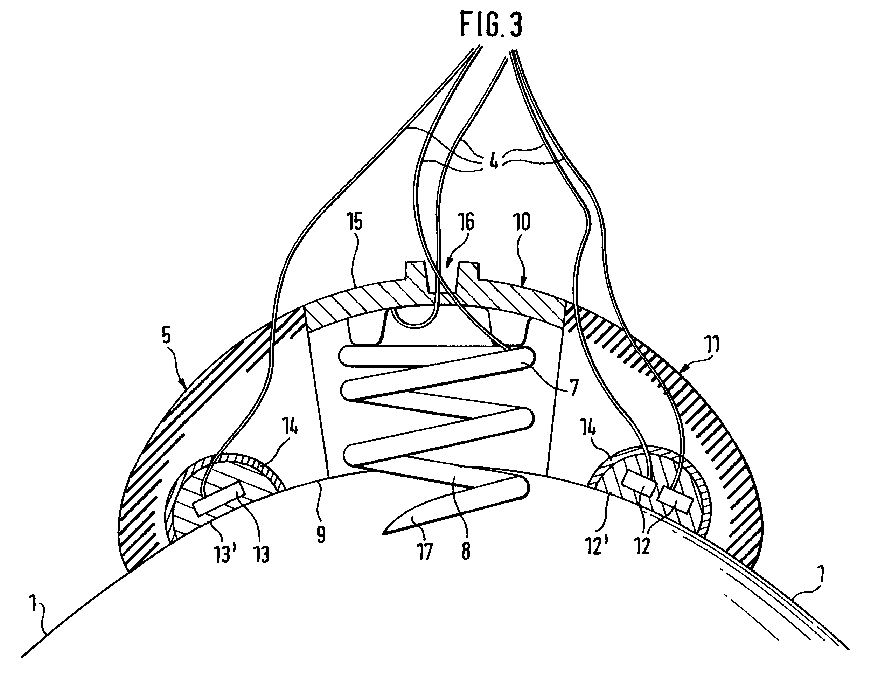

Wie aus den Fig. 2 und 3 ersichtlich, ist die Schale 5 auf ihrer konvexen Seite mit einer Metallplatte 15 versehen, die zum Teil im Werkstoff der Schale 5 eingebettet ist und dort mit der Drahtspirale 7 fest verbunden ist, wobei jedoch Metallplatte und Spirale gegeneinander elektrisch isoliert sind.

Die Metallplatte 15 bildet eine Baueinheit mit einem im Zentrum angeordneten Innen-Mehrkant 16, das als Kupplungsteil für eine Dreh-Handhabe dient.

Damit die Leitungen 4 auf die Schale 5 kein Drehmoment ausüben können, sei es beim Befestigen, sei es während der Geburt, können die Leitungen 4 durch den Kupplungsteil, also im Zentrum der Schale 5, durchgeführt werden. Die Drehhandhabe kann dazu am vorderen Ende zum Beispiel als Gabel ausgebildet werden, wobei die Leitungen 4 seitlich in die Gabel eingeführt sind.

Zu der Metallplatte 15 und der Drahtspirale 7 führt jeweils eine gesonderte Leitung 4, so daß diese Bauteile als EKG-Elektroden dienen können.

Der Lichtemitter 12 und der Empfänger 13 ist mit dem Meßgerät 3 jeweils mit eigenen Leitungen 4 verbunden.

In Figur 6 ist das Ende 17 der Drahtspirale 7 dargestellt. Das Ende 17 ist mit einer Schräge 18 versehen, die zur Bildung einer kleinen Schneidkante zum Kopf 1 des Feten hinweist.

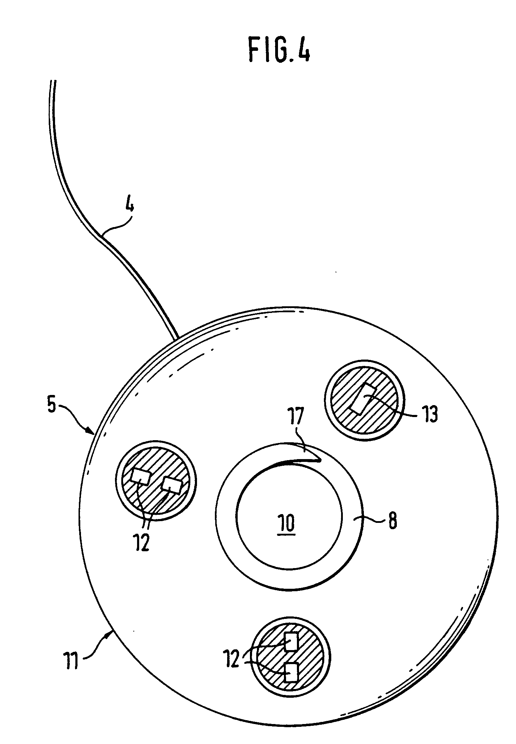

In Figur 4 ist eine Anordnung von zwei Lichtemittern 12 und einem Empfänger 13 gezeigt, die jeweils auf dem Eckpunkt eines Dreiecks liegen.

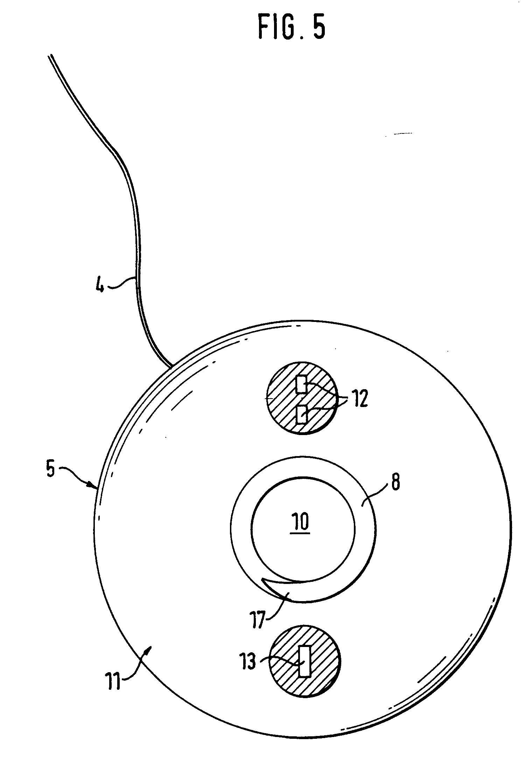

Jeder Lichtemitter 12 und der Empfänger 13 ist also zum Zentrum der Schale 5 etwa gegenüberliegend, aber in einem Winkel ungleich 180° angeordnet, wie dies in Figur 5 bei einem Lichtemitter 12 und einem Empfänger 13 gezeigt ist.

In einem bevorzugten Ausführungsbeispiel beträgt der Durchmesser der Schale 5 etwa 14 mm und die Dicke der Schale im Zentrum etwa 2 mm, während die Shorehärte etwa 30 Shore beträgt.

Die Elastizität der Randzone 11 und ihre Anschmiegsamkeit kann durch die Wahl der Shorehärte des Werkstoffes, auch durch eine Änderung der Dicke der Schale und evtl. durch ihre Verjüngung im erforderlichen Maß erzielt werden.

Die Arbeits- und Funktionsweise der Sensoreinrichtung wird nachfolgend erläutert.

Der Träger 2 kann infolge seiner geringen Dimension und seiner einfachen Handhabung bereits in einem frühen Stadium der Geburt durch die Vagina eingeführt und am führenden Teil des Feten befestigt werden.

Dazu wird der Träger 2 mit dem Zentrum leicht auf den Kopf des Feten gedrückt, z.B.mittels einer Dreh-Handhabe, die an ihrem vorderen Ende ein Mehrkantprofil aufweist, das in das Mehrkantprofil 16 der Schale 5 paßt.

Durch eine Umdrehung des Trägers 2 um etwa 360° wird die Drahtspirale 7 in das Gewebe des Feten eingedreht. Nachdem die Spirale 7 nur mit einer Windung aus der Fläche 9 der Höhlung 6 ragt, wird durch eine volle Umdrehung des Trägers 2 gewährleistet, daß die Windung der Spirale 7 in das Gewebe eingedreht ist, ohne eine Beeinträchtigung des Gewebes zu verursachen. Dabei hilft auch die Schräge 18 am Ende der Spirale 7, daß das so angeschärfte Ende sofort in das Gewebe eindringt, wenn die Spirale 7 auf das Gewebe drückt und anschließend gedreht wird.

Die Drehkraft wird dabei über das Mehrkantprofil und die Metallplatte 15 unmittelbar auf die damit fest verbundene Spirale 7 übertragen.

Wenn der Träger 2 mit seinem Zentrum auf das Gewebe gedrückt wird, kommt als erstes die Randzone 11 der Schale 5 mit dem Gewebe in Berührung. Die Randzone 11 verformt sich dann elastisch, die Krümmung der Schale 5 verringert sich. Wenn die Spirale 7 in das Gewebe eingedreht ist, liegt die Randzone 11 der Schale 5 mit leichter Vorspannung auf dem Gewebe auf, so daß auch Lichtemitter 12 und Empfänger 13 mit ihrer Oberfläche am Gewebe des Feten anliegen.

Die Befestigungszone 10 wird durch die Spirale 7 ebenfalls an das Gewebe gedrückt. Das Andrücken des Trägers 2 an das Gewebe erfolgt auf diese Art und Weise auch im Zentrum sehr schonend und dicht, aber auf jeden Fall wird vermieden, daß im Bereich der Randzone 11 der arterielle Blutfluß des Feten beeinträchtigt wird.

Vom Emitter 12 wird das Licht in das Gewebe des Feten geschickt, wo es sich nach allen Richtungen ausbreitet, so daß ein Teil des Lichtes auch auf den Empfänger 13 trifft. Die beste Modulation wird dabei erzielt, wenn Lichtemitter und Empfänger wie in den Figuren 4 oder 5 angeordnet sind. Das Licht breitet sich aber im Gewebe des Feten nach allen Richtungen aus, (anders als in anderen Medien), so daß noch genügend Licht auf den Empfänger 13 treffen würde, wenn Emitter und Empfänger diametral gegenüber angeordnet sein würden.

Von Bedeutung bezüglich der Lichtausbeute ist es auch, Lichtverluste zu vermeiden. Vor allem sollte verhindert werden, daß Licht im Kurzschluß vom Emitter zum Empfänger gelangen kann, ohne den Weg durch das Gewebe des Feten zu nehmen.

Dieser Nachteil kann vermieden werden, indem der Werkstoff der Schale 5 eine Farbe aufweist, die für die verwendeten Lichtwellen undurchlässig ist oder sie absorbiert.

Weder kann dann Licht in den Werkstoff der Schale 5 gelangen, noch kann der Kurzschluß-Effekt eintreten, wenn zwischen der Oberfläche des Emitters bzw. Empfängers und dem Gewebe ein kleiner Spalt verbleiben würde.

Die gleiche Wirkung kann erzielt werden, indem Emitter und Empfänger nach rückwärts durch eine Kappe 14 optisch abgeschirmt sind und die Fläche 9 der Höhlung 6 mit einer Farbe versehen ist, welche die verwendeten Lichtwellen absorbiert.

Wie in Figur 1 gezeigt, kann jeweils im Bereich eines Emitters 12 und/oder eines Empfängers 13 ein lappenförmiges Ohr 19 angebracht sein, das die Schale 5 radial nach außen überragt und das Gewebe des Feten in diesem Bereich gegen Lichtaustritt bzw. -Eintritt abdeckt. Im Gewebe des Feten breitet sich das Licht nämlich in einem Bereich von etwa 8 mm radial über den Bereich der Schale aus. Es ist deshalb vorteilhaft, diesen Bereich mit Ohren 19 abzudecken.

Die Befestigung des Trägers 2 am Gewebe des Feten kann auch dadurch erfolgen, daß das Zentrum der Schale 5 am Gewebe angeklebt wird.

Auch andere Befestigungsarten können verwendet werden, wenn nur gewährleistet ist, daß die Randzone 11 der Schale beim Andrücken des Trägers an das Gewebe elastisch federnd zurückweichen kann und unter leichter Vorspannung am Gewebe anliegt.

Die Befestigung des Trägers mittels der Drahtspirale bringt allerdings die Möglichkeit, daß die elektrisch gegeneinander isolierten Teile, nämlich Metallplatte 15 und Spirale 7 als Elektroden verwendet werden können, beispielsweise als EKG-Elekrode.

Hervorzuheben ist die besonders einfache Bauweise des Trägers und seine sehr kleine Form.

Er ist einfach und wirtschaftlich (als Verbrauchsartikel) herzustellen.

Dennoch erfüllt er die Aufgabe einer sicheren, dauerhaften Befestigung am Gewebe und eines einwandfreien Erzielens von Signalen für die Messung von vitalen Parametern eines Feten während der Geburt in hervorragender Weise.

## Patentansprüche

1. Sensoreinrichtung als Teil einer Meßvorrichtung mit einem Meßgerät zum Messen von vitalen Parametern eines Feten während der Geburt, insbesondere des Sauerstoffgehalts des Blutes des Feten, wobei die Sensoreinrichtung einen im Querschnitt etwa runden Träger mit einer Höhlung aufweist, in welcher etwa zentrisch ein Befestigungsmittel zum Befestigen des Trägers am führenden Teil des Feten angeordnet ist und der Träger (2) als eine gekrümmte Schalte (5) ausgebildet ist, die auf ihrer konkaven Seite die Höhlung (6) bildet,
dadurch gekennnzeichnet,
daß die gekrümmte Schalte (5) in eine im Zentrum befindliche Befestigungszone (10) und in eine diese umgebende elastische Randzone (11) unterteilt ist in welcher mindestens ein Lichtemitter (12) und mindestens ein Empfänger (13) angeordnet sind, wobei die Randzone als Federmittel ausgebildet ist, wodurch die Randzone (11) der am Gewebe befestigten Schale (5) unter federnder Vorspannung nachgiebig auf das Gewebe des Feten drückt.

2. Sensoreinrichtung nach Anspruch 1, **dadurch gekennzeichnet**, daß das Befestigungsmittel als Drahtspirale (7) derart ausgebildet und angeordnet ist, daß sie mit einem beliebig geformten Teil im Werkstoff der Schale (5) eingebettet ist und der andere als Spirale (7) ausgebildete Teil mit etwa einer Windung aus der Fläche (9) der Höhlung (6) ragt.

3. Sensoreinrichtung nach Anspruch 1, **dadurch gekennzeichnet,** daß das Befestigungsmittel durch einen Klebstoff dargestellt ist.

4. Sensoreinrichtung nach mindestens einem der Ansprüche 1 bis 3, **dadurch gekennzeichnet,** daß Lichtemitter (12) und Empfänger (13) zum Teil im Werkstoff der Schale (5) eingebettet sind und ihre Oberfläche etwa bündig mit der Fläche (9) der Höhlung (6) verläuft.

5. Sensoreinrichtung nach mindestens einem der Ansprüche 1 bis 4, **dadurch gekennzeichnet,** daß der Werkstoff der Schale (5) eine Farbe aufweist, die für die verwendeten Lichtwellen undurchlässig ist oder sie absorbiert.

6. Sensoreinrichtung nach mindestens einem der Ansprüche 1 bis 5, **dadurch gekennzeichnet,** daß Lichtemitter (12) und Empfänger (13) innerhalb der Schale (5) nach rückwärts abgeschirmt sind und die Fläche (9) der Höhlung (6) eine Farbe aufweist, welche die verwendeten Lichtwellen absorbiert.

7. Sensoreinrichtung nach mindestens einem der Ansprüche 1 bis 6, **dadurch gekennzeichnet,** daß Lichtemitter (12) und Empfänger (13) zum Zentrum der Schale (5) etwa gegenüberliegend, aber in einem Winkel von ungleich 180° angeordnet sind.

8. Sensoreinrichtung nach Anspruch 7, **dadurch gekennzeichnet,** daß zwei Lichtemitter (12) und ein Empfänger (13) vorgesehen sind, die jeweils etwa auf einem Eckpunkt eines Dreiecks liegen.

9. Sensoreinrichtung nach mindestens einem der Ansprüche 1 bis 8, **dadurch gekennzeichnet,** daß der Durchmesser der Schale etwa 14 mm, die Dicke der Schale etwa 2 mm und die Shore-Härte des Werkstoffes der Schale etwa 30 ShoreHärte betragen.

10. Sensoreinrichtung nach mindestens einem der Ansprüche 2 und 4 bis 9, **dadurch gekennzeichnet,** daß die Schale (5) auf ihrer konvexen Seite im Zentrum mit einem Kupplungsteil für eine Dreh-Handhabe versehen ist.

11. Sensoreinrichtung nach Anspruch 10, **dadurch gekennzeichnet,** daß der Kupplungsteil als Innen-Mehrkant (16) ausgebildet ist.

12. Sensoreinrichtung nach einem der Ansprüche 10 und 11, **dadurch gekennzeichnet**, daß der Kupplungsteil eine Baueinheit mit einer Metallplatte (15) bildet, die zum Teil im Werkstoff der Schale (5) eingebettet und dort mit der Drahtspirale (7) fest verbunden ist.

13. Sensoreinrichtung nach Anspruch 12, **dadurch gekennzeichnet,** daß Metallplatte (15) und Drahtspirale (7) gegeneinander elektrisch isoliert und jeweils mit einer eigenen elektrischen Leitung (4) versehen sind.

14. Sensoreinrichtung nach mindestens einem der Ansprüche 2 und 4 bis 13, **dadurch gekennzeichnet,** daß das freie Ende der Drahtspirale (7) so angeschärft ist, daß die angeschliffene Fläche (18) zur Schale (5) hin weist.

15. Sensoreinrichtung nach mindestens einem der Ansprüche 1 bis 14, **dadurch gekennzeichnet,** daß jeweils im Bereich eines Emitters (12) und/oder eines Empfängers (13) an der Schale (5) ein diese radial nach außen überragendes lappenförmiges Ohr (19) angebracht ist.

16. Sensoreinrichtung nach Anspruch 10 oder 13, **dadurch gekennzeichnet,** daß die Leitungen (4) durch den im Zentrum befindlichen Kupplungsteil geführt sind.

## Claims

1. Sensor device as part of a measurement device with a measurement unit for measuring vital parameters of a fetus during labor and delivery, especially the oxygen content of the fetal blood, wherein the sensor device has a carrier with an approximately round cross-section and with a concavity in which an attachment device is arranged approximately centrally for attaching the carrier to the presenting part of the fetus and the carrier (2) is designed as a curved cup (5) forming the concavity (6) on its concave side, **characterized in** that the curved cup (5) is subdivided into an attachment zone (10) at the center and an elastic peripheral zone (11) surrounding the same, in which at least one light emitter (12) and at least one receiver (13) are arranged, said peripheral zone being designed as a spring means, whereby the peripheral zone (11) of the cup (5) attached to the tissue is in flexible spring-loaded contact with the fetal tissue.

2. Sensor device according to Claim 1, **characterized in** that the attachment device is designed and arranged as a spiral wire (7) such that it is embedded with a part shaped in any desired form in the material of the cup (5) and the other part which is designed as a spiral (7) projects out of the surface (9) of the concavity (6) by about one turn.

3. Sensor device according to Claim 1, **characterized in** that the attachment device is represented by an adhesive.

4. Sensor device according to at least one of Claims 1 to 3, **characterized in** that light emitter (12) and receiver (13) are partially embedded in the material of the cup (5) and their surfaces are approximately flush with the surface (9) of the concavity (6).

5. Sensor device according to at least one of Claims 1 to 4, **characterized in** that the material of the cup (5) has a color that is impermeable to the light waves used or has a color that absorbs such light waves.

6. Sensor device according to at least one of Claims 1 to 5, **characterized in** that light emitter (12) and receiver (13) are shielded rearwards inside the cup (5), and the surface (9) of the concavity (6) has a color that absorbs the light waves used.

7. Sensor device according to at least one of Claims 1 to 6, **characterized in** that light emitter (12) and receiver (13) are approximately opposite each other relative to the center of the cup (5) but they are arranged at an angle not equal to 180°.

8. Sensor device according to Claim 7, **characterized in** that two light emitters (12) and one receiver (13) are provided that are each arranged approximately at a comer point of a triangle.

9. Sensor device according to at least one of Claims 1 to 8, **characterized in** that the diameter of the cup is approximately 14 mm, the thickness of the cup is approximately 2 mm and the Shore hardness of the material of the cup is approximately 30 Shore.

10. Sensor device according to at least one of Claims 2 and 4 to 9, **characterized in** that the cup (5) is provided with a coupling part for a rotating handle in the center of its convex side.

11. Sensor device according to Claim 10, **characterized in** that the coupling part is designed as an internal polygon (16).

12. Sensor device according to one of Claims 10 and 11, **characterized in** that the coupling part forms a structural unit with a metal plate (15) that is partially embedded in the material of the cup (5) where it is firmly connected to the spiral wire (7).

13. Sensor device according to Claim 12, **characterized in** that metal plate (15) and spiral wire (7) are electrically insulated with respect to each other and are each provided with their own electric line (4).

14. Sensor device according to at least one of Claims 2 and 4 to 13, **characterized in** that the free end of the spiral wire (7) is sharpened so that the sharpened surface (18) points toward the cup (5).

15. Sensor device according to at least one of Claims 1 to 14, **characterized in** that a tab-shaped ear (19) that projects radially outward beyond the cup (5) is mounted on the cup in the area of an emitter (12) and/or a receiver (13).

16. Sensor device according to Claims 10 or 13, **characterized in** that the lines (4) pass through the coupling part located in the center.

## Revendications

1. Capteur faisant partie d'un dispositif de mesure comportant un appareil de mesure destiné à mesurer les paramètres vitaux d'un foetus pendant l'accouchement, en particulier la quantité d'oxygène dans le sang du foetus, étant précisé que ce capteur comporte un support à section transversale à peu près ronde, qui présente un creux dans lequel un moyen de fixation destiné à fixer le support est disposé de façon centrée sur la partie du foetus qui se présente, et que le support (2) est conçu comme une coquille courbe (5) qui définit sur son côté concave le creux (6),
caractérisé en ce que la coquille courbe (5) est divisée en une zone de fixation (10) située au centre et une zone extérieure élastique (11) qui entoure celle-ci et dans laquelle sont disposés au moins un émetteur de lumière (12) et au moins un récepteur (13), la zone extérieure étant conçue comme un moyen formant ressort, moyennant quoi cette zone extérieure (11) de la coquille (5) fixée au tissu du foetus appuie de manière souple sur ledit tissu en étant soumise à une contrainte de ressort.

2. Capteur selon la revendication 1, caractérisé en ce que le moyen de fixation est conçu et disposé comme un fil métallique en spirale (7) de manière à être encastré avec une partie de forme quelconque dans la matière de la coquille (5), tandis que l'autre partie conçue comme une spirale (7) dépasse avec environ une spire de la surface (9) du creux (6).

3. Capteur selon la revendication 1, caractérisé en ce que le moyen de fixation est constitué par une colle.

4. Capteur selon l'une au moins des revendications 1 à 3, caractérisé en ce que l'émetteur de lumière (12) et le récepteur (13) sont en partie encastrés dans la matière de la coquille (5) et leur surface affleure à peu près la surface (9) du creux (6).

5. Capteur selon l'une au moins des revendications 1 à 4, caractérisé en ce que la matière de la coquille (5) présente une couleur qui ne laisse pas passer les ondes lumineuses utilisées, ou qui les absorbe.

6. Capteur selon l'une au moins des revendications 1 à 5, caractérisé en ce que l'émetteur de lumière (12) et le récepteur (13) sont blindés vers l'arrière, à l'intérieur de la coquille (5), et la surface (9) du creux (6) présente une couleur qui absorbe les ondes lumineuses utilisées.

7. Capteur selon l'une au moins des revendications 1 à 6, caractérisé en ce que l'émetteur de lumière (12) et le récepteur (13) sont disposés à peu près face à face par rapport au centre de la coquille (5), mais suivant un angle différent de 180°.

8. Capteur selon la revendication 7, caractérisé en ce qu'il est prévu deux émetteurs de lumière (12) et un récepteur (13) qui se trouvent chacun au niveau d'un sommet d'un triangle.

9. Capteur selon l'une au moins des revendications 1 à 8, caractérisé en ce que le diamètre de la coquille est d'environ 14 mm, son épaisseur d'environ 2 mm et la dureté Shore de sa matière d'environ 30.

10. Capteur selon l'une au moins des revendications 2 et 4 à 9, caractérisé en ce que la coquille (5) est pourvue, au centre de son côté convexe, d'un élément d'accouplement pour une poignée de rotation.

11. Capteur selon la revendication 10, caractérisé en ce que l'élément d'accouplement est conçu comme une empreinte polygonale (16).

12. Capteur selon la revendication 10 ou 11, caractérisé en ce que l'élément d'accouplement forme une unité de construction avec une plaque métallique (15), qui est en partie encastrée dans la matière de la coquille (5) et qui est reliée fermement, à cet endroit, au fil métallique en spirale (7).

13. Capteur selon la revendication 12, caractérisé en ce que la plaque métallique (15) et le fil métallique en spirale (7) sont isolés électriquement l'un par rapport à l'autre et sont pourvus chacun d'une ligne électrique (4) propre.

14. Capteur selon l'une au moins des revendications 2 et 4 à 13, caractérisé en ce que l'extrémité libre du fil métallique en spirale (7) est affûtée de telle sorte que la surface affûtée (18) soit dirigée vers la coquille (5).

15. Capteur selon l'une au moins des revendications 1 à 14, caractérisé en ce qu'il est prévu dans la zone d'un émetteur (12) et/ou d'un récepteur (13), sur la coquille (5), une oreille en forme de patte (19) qui dépasse de ladite coquille (5) radialement vers l'extérieur.

16. Capteur selon la revendication 10 ou 13, caractérisé en ce que les lignes (4) traversent l'élément d'accouplement situé au centre.
